# EUROPÄISCHE PATENTSCHRIFT

(11) **EP 2 504 691 B1**
(45) Veröffentlichungstag und Bekanntmachung des Hinweises auf die Patenterteilung: **07.09.2016**
(21) Anmeldenummer: 10788008.0
(22) Anmeldetag: 23.11.2010
(51) Int. Cl.: G01N 27/12

(54) **VERFAHREN ZUM HERSTELLEN EINES HEIZBAREN GASSENSORS SOWIE VERWENDUNG EINES STÜTZMITTELS ZU DESSEN HERSTELLUNG**
METHOD FOR MANUFACTURING A HEATABLE GAS SENSOR AND USAGE OF A SUPPORTING STRUCTURE FOR ITS MANUFACTURE
PROCEDE POUR LA PRODUCTION D'UN CAPTEUR DE GAZ POUVANT ETRE CHAUFFE ET UTILISATION D'UNE STRUCTURE DE SUPPORT POUR SA PRODUCTION

(30) Priorität: 25.11.2009 DE 102009054435
(43) Veröffentlichungstag der Anmeldung: 03.10.2012
(73) Patentinhaber: Wiesner, Manfred, 56170 Bendorf (DE)
(72) Erfinder: Wiesner, Manfred, 56170 Bendorf (DE)
(74) Vertreter: Grosse Schumacher Knauer von Hirschhausen
(86) Internationale Anmeldenummer: PCT/EP2010/007077
(87) Internationale Veröffentlichungsnummer: WO 2011/063925

(56) Entgegenhaltungen:
- DE-A1- 2 005 497
- DE-A1- 19 924 611
- JP-A- S6 191 555
- JP-A- 2005 227 002
- US-A- 3 865 550
- US-A- 3 955 268
- US-A- 4 596 975
- US-A- 4 723 439
- US-A- 4 731 226
- US-A- 4 755 473
- US-B1- 6 319 473
- "Leitfähigkeitssensoren" In: "Sensoren - Prinzipien und Anwendungen", 1 January 1991 (1991-01-01), Carl Hanser Verlag, München Wien, XP055150067, ISBN: 978-3-44-616073-6 pages 105-109, * page 105 - page 109 *

## Beschreibung

### GEBIET DER ERFINDUNG

Die Erfindung betrifft ein Verfahren zum Herstellen eines heizbaren Gassensors und eine Verwendung eines Stützmittels nach dem Oberbegriff des Anspruchs 2 bzw. 1.

### TECHNISCHER HINTERGRUND

Zur Detektion und ggf. Bestimmung der Konzentration von Stoffen wie etwa Methan, Ethan, Propan, Kohlenmonoxid, Wasserstoff etc. in Gasgemischen, Heizgasen, Abgasen und/oder dergleichen sind aus dem Stand der Technik Gassensoren auf Basis anorganischer Metalloxid-Halbleiter (MOX) bekannt. Bei Kontakt derartiger als poröser Festkörper ausgebildeter Gassensoren mit den vorgenannten oder anderen reduzierenden Stoffen ändert sich die Leitfähigkeit des Metalloxids jedenfalls an dessen Oberfläche. Die Änderung der Leitfähigkeit des Metalloxids kann mittels einer Elektronik erfasst und ausgewertet werden, um reduzierende Stoffe zu detektieren und ggf. in ihrer Konzentration zu bestimmen.

Ein derartiger Gassensor ist aus der DE 44 28 155 C2 bekannt. Der Gassensor umfasst ein Substrat, auf dem eine interdigitale Elektrodenstruktur in Form von zwei kammartig ineinander greifenden, aber voneinander beabstandeten elektrischen Elementen ausgebildet ist. Auf der interdigitalen Elektrodenstruktur und in Kontakt mit dieser ist eine Schicht aus gassensitivem Metalloxid, hier Galliumoxid, aufgebracht. Bei Kontakt mit einem brennbaren Gas ändert sich die Leitfähigkeit des Metalloxids und eine Gaskonzentration kann dementsprechend durch Messen des elektrischen Widerstands zwischen den beiden voneinander beabstandeten kammartig ineinander greifenden elektrischen Elementen der interdigitalen Elektrodenstruktur bestimmt werden.

Die Leitfähigkeitsänderung des gassensitiven Metalloxids ist jedoch temperaturabhängig. Daher ist bei der DE 44 28 155 C2 ein Temperaturfühler auf dem Substrat benachbart zur interdigitalen Elektrodenstruktur als separates Element vorgesehen. Die tatsächliche Temperatur des Gassensors muss also ermittelt und bei der Auswertung der gemessenen Widerstandswerte berücksichtigt werden.

Bei zu geringen Temperaturen ist zudem der aus der DE 44 28 155 C2 bekannte Gassensor nicht einsetzbar, da ein die Leitfähigkeitsänderung bewirkender Elektronenübergang der adsorbierten Moleküle der reduzierenden Stoffe bei Galliumoxid erst bei Temperaturen oberhalb von 450°C in sinnvoll auswertbarer Quantität stattfindet. So ist Galliumoxid bei Temperaturen über etwa 450°C ein n-Halbleiter. Im Bereich zwischen etwa 450°C und 700°C weist Galliumoxid eine starke Sensitivität gegenüber reduzierenden Stoffen wie Methan, Ethan, Propan, Kohlenmonoxid, Wasserstoff und dergleichen auf, die eine Folge von Oberflächenwechselwirkungen ist. Daher ist der bekannte Gassensor auf Einsatzzwecke beschränkt, in denen das zu überwachende Gas eine ausreichend hohe Temperatur aufweist. Dies ist beispielsweise bei Abgasen von Verbrennungsprozessen der Fall.

Um einen Gassensor auf Metalloxid-Basis auch bei niedrigeren Umgebungstemperaturen einsetzen zu können, ist es aus der EP 0 464 244 B1 bekannt, auf einem Substrat, das kammartig ineinander greifende und voneinander beabstandete elektrische Elemente zum Messen der Leitfähigkeit des Metalloxids aufweist, auch ein elektrisches Element zum Heizen vorzusehen. Das elektrische Element zum Heizen ist in Form einer mäanderförmigen Leiterbahn ausgeführt, die mit Heizstrom beaufschlagbar ist und sich auf der den elektrischen Elementen zum Messen der Leitfähigkeit des Metalloxids entgegengesetzten Oberfläche des Substrats befinden. Damit ist ein Aufheizen des Metalloxids, das auch hier als Galliumoxid ausgebildet ist, auf eine Temperatur oberhalb von 450°C möglich.

Durch die Ausbildung der elektrischen Elemente zum Heizen des Metalloxids auf einer Seite eines Substrats und zum Messen einer Leitfähigkeit des Metalloxids auf der gegenüber liegenden Seite des Substrats ergeben sich bei dem aus der EP 0 464 244 B1 bekannten Gassensor Nachteile. So ist einerseits die Fertigung aufwendig und teuer. Andererseits wirkt die Heizenergie nicht direkt auf das die elektrischen Elemente zum Messen des Metalloxids umgebende Metalloxid, sondern durch das Substrat hindurch. Schließlich ist die Sensitivität des Sensors nicht optimal, da sich zwischen den elektrischen Elementen zum Messen des Metalloxids nur eine begrenzte Menge Metalloxid befindet, und der Sensor ist nur einseitig gassensitiv.

Da die Sensoren unter typischen Einsatzbedingungen hohen Temperaturen bis zu 1200°C, mechanischen Belastungen in Form von Schwingungen und thermischen Spannungen durch extreme Temperaturwechsel ausgesetzt sind und zudem chemischen Einflüssen durch reduzierende und korrodierende Gasbestandteile über längeren Zeitraum ohne merkliche Alterung standhalten müssen, ergeben sich bei den bekannten substratbasierten Sensoren prinzipbedingt Schwächen, die durch erhöhten technischen Aufwand kompensiert werden müssen. So ist bei dem aus der EP 0 464 244 B1 bekannten Gassensor zur Vermeidung katalytischer Reaktionen an den offenen Flächen der Heizanordnung eine Passivierung mittels Kieselsäure erforderlich. Bei einer Verdampfung der Passivierungsschicht in Folge der vorgenannten widrigen Betriebsbedingungen ergeben sich nachteilige Auswirkungen auf die Sensitivität des Gassensors. Zudem kann das Substrat zu Schwingungen angeregt werden und thermische Spannungen können zur Beschädigung der elektrischen Elemente auf dem Substrat führen.

Schließlich ist die Herstellung eines substratbasierten Gassensors, wie er aus der DE 44 28 155 C2 oder der EP 0 464 144 B1 bekannt ist, technisch aufwendig und teuer, da spezielle Vorrichtungen für die Ausbildung kleiner Strukturen auf einem Substrat erforderlich sind.

Ein substratfreier Gassensor auf Basis eines anorganischen Metalloxid-Halbleiters ist zwar aus der US 3 835 529 bekannt. Allerdings ist in der substratfreien Ausführungsform ein Heizelement zum Heizen des Metalloxids nicht vorgesehen. Bei Vorhandensein eines Heizelements ist dieses auf einem Träger aufgebracht, der ein Substrat darstellt, welches zudem mit erhitzt werden muss. Hierdurch ergeben sich die bereits vorgenannten Nachteile. Zudem ist bei den aus der US 3 835 529 bekannten Gassensor zwingend ein zylinderförmiges gasundurchlässiges Metallgehäuse erforderlich, da das Metalloxid in loser Pulverform vorliegt. Das gasundurchlässige Gehäuse verringert die Sensibilität des Sensors, da Gas nicht allseitig den Sensor erfassen kann, sondern durch stirnseitige Endkappen den Sensor durchströmen muss. Zudem ergibt sich bei einer Heizung des Gassensors eine ungünstige Eigenthermik. Schließlich wirkt sich die lose Schüttung des Metalloxids nachteilig auf die Signalstabilität aus, da mechanische Belastungen wie etwa Schwingungen das Kontaktgefüge stören.

Bei dem aus der DE 10 2008 055 568 A1 bekannten Gassensor sind die elektrischen Elemente zum Heizen getrennt von den elektrischen Elementen zum Messen ausgebildet. Gemeinsam zum Heizen und Messen benutzte elektrische Elemente sind nicht vorgesehen und auch nicht räumlich in Metalloxid eingebettet. Vielmehr lehrt die DE 10 2008 055 568 A1 einen klassischen Schichtaufbau.

Bei dem aus der DE 198 59 998 A1 bekannten Gassensor sind die Messelemente auf einem Substrat angeordnet. Grundsätzlich ist dies problematisch, da Fehlströme entstehen können. Zwischen den Messelementen ist das Substrat vertieft und die über die Messelemente aufgebrachte Metalloxidschicht erstreckt sich bis in die Vertiefung hinein. Eine von den Messelementen separat ausgebildete Heizung ist von der sensitiven Schicht entkoppelt.

Der Gassensor der DE 44 01 570 A1 verfügt über kein Heizelement, ist nicht allseitig empfindlich und benötigt eine von einer Halbleiterschicht isolierte dritte Elektrode.

Aus der DE 690 18 742 T2 sind schließlich ein Thermistor und ein Gassensor mit diesem Thermistor bekannt, wobei die Heizung vom Thermistor getrennt ausgebildet ist. Ferner weist der Thermistor kein poröses Material auf und der Gassensor ist nicht allseitig empfindlich.

US 3,865,550 und US 3,955,268 und JP2005227002 und JP61091555 offenbaren jeweils Gassensoren mit einem gassensitiven porösen Metalloxid mit zwei helixartig um eine gemeinsame Achse gewundenen elektrischen Elementen, die zum Heizen und zum Messen einer Leitfähigkeit des Metalloxids ausgestaltet sind.

Ausgehend hiervon liegt der Erfindung die Aufgabe zugrunde, ein Verfahren zur Herstellung eines heizbaren Gassensors und eine Verwendung eines Stützmittels nach dem Oberbegriff des Anspruchs 2 bzw. 1 zu schaffen, die bei erhöhter Sensitivität und herstellungskostensenkender Vereinfachung des Aufbaus eine erhöhte Einsatzdauer auch unter widrigen Einsatzbedingungen ermöglichen.

### DARSTELLUNG DER ERFINDUNG

Diese Aufgabe wird durch eine Verwendung und ein Verfahren mit den Merkmalen des Anspruchs 1 bzw. 2 gelöst.

Demnach wird ein Verfahren zu Herstellung eines heizbaren Gassensors für Gase, die reduzierende Stoffe wie etwa Methan, Ethan, Propan, Kohlenmonoxid, Wasserstoff und/oder dergleichen enthalten, mit einem gassensitiven porösen Metalloxid sowie voneinander beabstandeten elektrischen Elementen zum Heizen des Metalloxids und zum Messen einer Leitfähigkeit des Metalloxids geschaffen, bei dem die Elemente jeweils räumlich im gesinterten Metalloxid eingebettet sind.

Der so hergestellte Gassensor ist substratfrei und das gesinterte Metalloxid umgibt die elektrischen Elemente allseitig.

Hierdurch ergibt sich zunächst eine erhöhte Sensitivität, da die Leitfähigkeit in einem dreidimensionalen räumlichen Bereich entlang und zwischen den elektrischen Elementen gemessen wird, der im Vergleich zum bekannten zweidimensionalen Schichtaufbau eine wesentlich größere innere Oberfläche des porösen Metalloxids bietet, an der Reaktionen mit den reduzierenden Stoffen stattfinden können.

Zudem ist eine Richtungsabhängigkeit nicht mehr gegeben. Vielmehr ist der erfindungsgemäß hergestellte Gassensor allseitig gasempfindlich und muss nicht ausgerichtet werden.

Ferner werden thermische Spannungen in Folge Temperaturwechsel weitestgehend vermieden, da lediglich die elektrischen Elemente und das Metalloxid, nicht jedoch ein Substrat oder ein Heizkern und etwaige weitere Schichten und Elemente wie etwa ein Gehäuse mit jeweils unterschiedlichen Wärmeausdehnungskoeffizienten vorgesehen sind.

Erfindungsgemäß gestaltet sich die Herstellung eines derartigen Sensors einfacher und kostengünstiger, da weder eine Substratbearbeitung noch ein Gehäuse erforderlich sind.

Die kombinierte Verwendung der elektrischen Elemente sowohl zum Heizen als auch zum Messen ergibt eine Heizwirkung direkt am Messort und vereinfacht den Aufbau und die Herstellung, da eine separate Heizung nicht vorgesehen werden muss. So kann ein eingebettetes Element heizen und mit einem weiteren ebenfalls eingebetteten Element zur Messung eines Stroms oder einer Spannung dazwischen verwendet werden.

Schließlich erhöht das Sintern des Metalloxids sowohl die mechanische als auch die Signalstabilität des Gassensors. Das Sensorsignal bleibt zudem langzeitstabil unabhängig von den von außen einwirkenden mechanischen Einflüssen.

Das gassensitive Metalloxid umfasst hierbei vorzugsweise Galliumoxid, Aluminiumoxid oder eine Mischung aus Galliumoxid und Aluminiumoxid, wobei jedoch auch andere Metalloxide verwendbar sind. Wichtig ist eine ausreichende Porosität, damit eine messbare Oberflächenwechselwirkung zwischen reduzierendem Stoff und Metalloxid stattfinden kann. Das Maß der Porosität ist in Abhängigkeit von der Dicke des Metalloxids und insbesondere des Abstands der elektrischen Elemente zum Messen der Leitfähigkeit auf übliche Weise bestimmbar. Vorzugsweise liegt die offene Porosität bei 10 bis 75 % und insbesondere zwischen 30 % und 50 % und vorzugsweise um etwa 50 %. Die Bestimmung der offenen Porosität der gesinterten Proben wird mit dem Wassereindringverfahren nach DIN EN 623 Teil 2 vorgenommen. Das Prinzip beruht darauf, dass ein poröser Körper eine Massenzunahme erfährt, wenn seine von außen zugänglichen Poren mit Flüssigkeit gefüllt werden. Die offene Porosität ist dimensionslos, und kann deshalb in Prozent angegeben werden. Eine offene Porosität von 50 % bedeutet beispielsweise, dass 50 % des Körpers mit offenen Poren gefüllt sind. Bei einer Reindichte von 2,7 g/cm³ und einer offenen Porosität von 50 % würde sich vereinfacht eine Rohdichte von 1,35 g/cm³ ergeben.

Zweckmäßigerweise ist wenigstens eines der Elemente zum Heizen des Metalloxids mit elektrischer Energie beaufschlagbar. Damit kann das Element mit einem Heizstrom beaufschlagt werden, der in Folge des Widerstands des elektrischen Elements zu einer Erhitzung führt. Die Erhitzung findet dabei unmittelbar in dem Bereich des Metalloxids statt, der benachbart zum Element sich befindet. Hierdurch kann der räumliche Bereich des porösen Metalloxids, in dem die Leitfähigkeitsänderung gemessen wird, unmittelbar geheizt werden; Fremdkörper wie etwa Substrate oder Heizelementträger sind nicht vorhanden und müssen daher weder geheizt werden, noch beschränken sie den räumlichen Messbereich. Es ergibt sich eine gute thermische Stabilität. Da das Messergebnis entscheidend von der Temperatur des Metalloxids abhängt, führt dies auch zu einer Verbesserung der Stabilität der Messung.

Ein weiteres elektrisches Element, das beabstandet zu dem zum Heizen des Metalloxids mit elektrischer Energie beaufschlagbaren Element angeordnet ist, kann verwendet werden, um die Leitfähigkeit des Metalloxids zwischen den beiden Elementen zu messen. Hierzu kann der Widerstand zwischen dem wenigstens einen zum Heizen des Metalloxids mit elektrischer Energie beaufschlagbaren Element und dem weiteren Element gemessen werden. Das mit Energie beaufschlagbare Element dient demnach sowohl zum Heizen des Metalloxids, als auch zum Messen der Leitfähigkeit.

Auch das weitere der Elemente kann zusätzlich zum Heizen des Metalloxids mit elektrischer Energie beaufschlagbar sein.

Die elektrischen Elemente können demnach sowohl zum Heizen als auch zum Messen verwendet werden. Beispielsweise können zwei voneinander beabstandete elektrische Elemente vorgesehen sein, die jeweils zwei nach außen geführte elektrische Anschlüsse aufweisen. In diesem Fall können beide elektrische Elemente mit elektrischer Energie zum Heizen des Metalloxids beaufschlagt werden und es ist möglich, die Leitfähigkeit des Metalloxids zwischen den beiden Elementen zu messen. Es kann auch vorgesehen sein, dass eines der Elemente mit zwei nach außen geführten elektrischen Anschlüssen vorgesehen ist, während ein weiteres lediglich einen Anschluss aufweist. Auch kann es vorgesehen sein, mehr als zwei elektrische Elemente vorzusehen.

Vorzugsweise erstrecken sich wenigstens zwei Elemente im Metalloxid zumindest abschnittsweise parallel, mäanderförmig, spiralförmig und/oder doppelhelixförmig beabstandet zueinander. Mit derartigen Strukturen kann der zur Messung der Leitfähigkeit herangezogene räumliche Bereich des Metalloxids zwischen den Elementen vergrößert werden.

Die Erfindung schafft ein Verfahren zum Herstellen eines heizbaren Gassensors, insbesondere wie vorstehend beschrieben, wobei wenigstens zwei elektrische Elemente zueinander beabstandet in gassensitivem porösem Metalloxid räumlich eingebettet werden, mit den Merkmalen des Anspruchs 2.

Hierdurch ergibt sich eine Vereinfachung des Herstellungsprozesses im Vergleich zu einem substratbasierten Gassensor, bei dem elektrische Strukturen auf einem Substrat aufgebracht und beschichtet werden müssen.

Ein Stützmittel sorgt dabei für den Abstand zwischen den beiden elektrischen Elementen. Das Stützmittel ist ein geeignetes Hilfsmittel zum Sicherstellen einer dreidimensionalen Struktur aus elektrischen Elementen. Das Stützmittel kann dabei eine Kammstruktur aufweisen und/oder bei der gemeinsamen Herstellung der elektrischen Elemente z.B. beim gemeinsamen Wickeln, Legen, Falten oder Formen oder dergleichen verwendet werden; auch kann es bei separater Herstellung der elektrischen Elemente beim Zusammenfügen derselben verwendet werden. Das Stützmittel kann nach dem gemeinsamen Ausbilden oder dem Zusammenfügen entfernt werden, noch bevor die gemeinsame Struktur in Metalloxid eingebettet wird; alternativ dazu kann das Stützmittel erst nach Einbetten in Metalloxid oder während des Einbettens entfernt werden. Jedenfalls stellt das Stützmittel sicher, dass eine dreidimensionale Struktur im Metalloxid eingebettet ist. Auch kann das Stützmittel nach Einlegen der elektrischen Strukturen in die Form und/oder Einbetten verwendet werden, um den gewünschten Abstand dazwischen herzustellen. Es kann neben der Stützfunktion auch eine Kalibrierfunktion erfüllen.

Die Herstellung kann mittels Spritz-, Press-, Gieß- oder Foliengussverfahren erfolgen. Bevorzugt ist es, die elektrischen Elemente in Metalloxidpulver einzubetten, wobei das Metalloxidpulver mit den eingebetteten Elementen im Anschluss in wenigstens einem Brand und ggf. zwei oder mehr Bränden gesintert wird. Vorzugsweise erfolgt das Sintern bei Temperaturen oberhalb von 1200°C und insbesondere bei Temperaturen in einem Bereich zwischen 1400°C und 1600°C, wobei die besten Ergebnisse im Bereich von 1400°C bis 1500°C erzielbar sind. Die Sintertemperatur und die Auswahl des Metalloxidpulvers hinsichtlich Körnigkeit und weiterer Eigenschaften und auch der vor dem Sintern auf das Metalloxidpulver ausgeübte Druck zum Verdichten sind im Einklang mit der Sintertemperatur und -dauer so zu wählen, dass sich die erforderliche offene Porosität unter Berücksichtigung der Dicke des Gassensors ergibt.

Besonders einfach ist die Herstellung, wenn zunächst Metalloxidpulver in einer Form eingebracht wird und auf die Schicht aus Metalloxidpulver mit Abstand zueinander die elektrischen Elemente aufgelegt werden. Die Elemente können dann mit einer weiteren Schicht Metalloxid bedeckt und dem Sinterprozess zugeführt werden.

Um den Abstand zwischen den Elementen kontrollieren zu können kann nach einer weiteren Ausführungsform der Erfindung es vorgesehen sein, zunächst Metalloxidpulver in eine Form einzubringen, ein Element, beispielsweise in Mäanderform, auf das Metalloxidpulver aufzulegen, das Element mit einer Schicht Metalloxidpulver zu bedecken, ein weiteres Element, beispielsweise ebenfalls mäanderförmig, aufzulegen und ebenfalls mit Metalloxidpulver zu bedecken, bevor gesintert wird.

Die vorgenannten sowie die beanspruchten und in den Ausführungsbeispielen beschriebenen erfindungsgemäß zu verwendenden Bauteile unterliegen in ihrer Größe, Formgestaltung, Materialauswahl und technischen Konzeption keinen besonderen Ausnahmebedingungen, so dass die in dem Anwendungsgebiet bekannten Auswahlkriterien uneingeschränkt Anwendung finden können.

Weitere Einzelheiten, Merkmale und Eigenschaften des Gegenstandes der Erfindung ergeben sich aus den Ansprüchen, sowie aus der nachfolgenden Beschreibung und den Figuren, in denen - beispielhaft - Ausführungsbeispiele dargestellt sind. Auch einzelne Merkmale der Ansprüche oder der Ausführungsformen können mit anderen Merkmalen anderer Ansprüche und Ausführungsformen kombiniert werden.

### KURZBESCHREIBUNG DER FIGUREN

Fig. 1(a), (b) und (c) illustriert einen Gassensor in Perspektivansicht, im Längsschnitt bzw. im Querschnitt.
Fig. 2 (a) und (b) illustriert eine weitere Ausführungsform eines Gassensors in Perspektivansicht bzw. im Längsschnitt.
Fig. 3(a), (b) und (c) illustrieren Schritte eines nicht erfindungsgemässen Verfahrens zum Herstellen eines Gassensors.
Fig. 4(a), (b) und Fig. 5(a), (b) illustrieren Schritte eines nicht erfindungsgemässen Verfahrens zum Herstellen eines Gassensors.
Fig. 6(a), (b), (c) und (d) illustriert Schritte eines erfindungsgemäßen Verfahrens zum Herstellen eines Gassensors in Draufsicht bzw. im Schnitt durch eine Form.
Fig. 7 illustriert eine weitere erfindungsgemässe Ausführungsform in Draufsicht.

### DETAILLIERTE BESCHREIBUNG VON AUSFÜHRUNGSBEISPIELEN

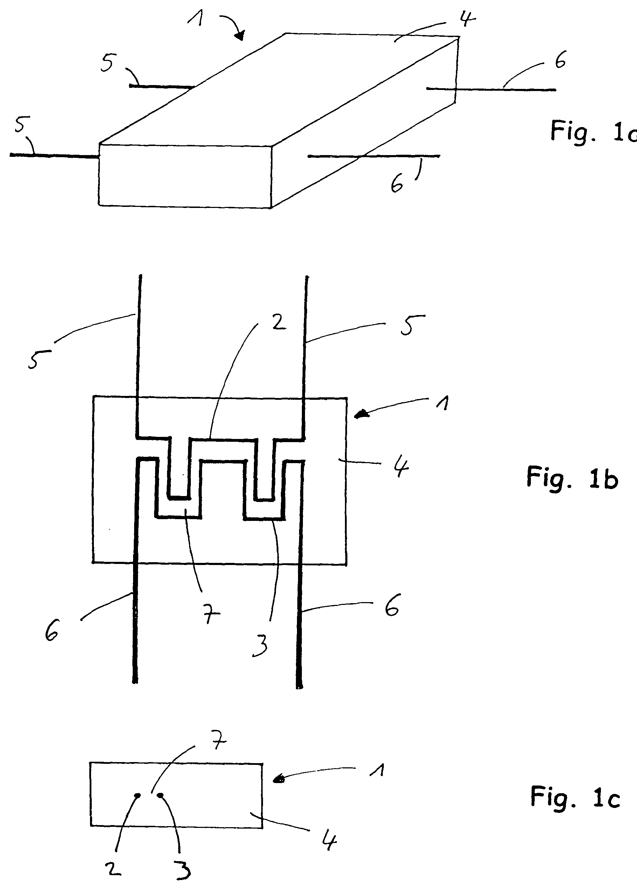

Der in Fig. 1 beispielhaft quaderförmig dargestellte Gassensor 1 weist zwei elektrische Elemente 2, 3 auf, die in einem Metalloxid 4 eingebettet und hier beispielhaft über seitlich aus dem Sensor 1 herausgeführte Anschlüsse 5, 6 elektrisch kontaktierbar sind.

Das Metalloxid, beispielsweise Galliumoxid, Aluminiumoxid oder eine Mischung daraus, ist vorzugsweise flach ausgeformt und kann eine Dimension von z. B. 7 mm X 2 mm X 7 mm aufweisen im Falle der beispielhaft dargestellten quaderförmigen Ausführung. In anderen Ausführungsformen kann das Metalloxid kugelförmig, eiförmig, münzförmig oder dergleichen sein.

Die in das Metalloxid eingebetteten, also allseitig vom Metalloxid umgebenen elektrischen Elemente 2, 3 können aus dünnem Metalldraht gebildet sein. Als Material für den Metalldraht eignet sich eine einfache Kupferlegierung, wobei jedoch der Korrosionsbeständigkeit in aggressiven Umgebungen wegen Edelmetalle wie Pt, An, Rh etc. bevorzugt werden, mit einem Durchmesser von beispielsweise 0,01 bis 1 mm und bevorzugt 0,1 bis 0,3 mm. Die beiden elektrischen Elemente 2, 3 weisen jeweils einen hier beispielhaft mäanderförmig gebildeten Abschnitt auf, in dem sie benachbart zueinander mit einem geringen Abstand von z. B. 0,1 bis 2 mm, insbesondere 0,1 bis 0,5 oder 1 mm verlaufen. Durch den Abstand wird ein dreidimensionaler Bereich 7 entlang und zwischen den elektrischen Elementen 2, 3 gebildet. Die Leitfähigkeit dieses dreidimensionalen Bereiches 7 ist ermittelbar, indem z. B. der Widerstand gemessen wird. Hierzu kann zwischen den Anschlüssen 5, 6 eine Spannung angelegt und der Stromfluss ermittelt werden. Die so gemessene Leitfähigkeit ist ein Maß für die Konzentration reduzierender Stoffe, die am Metalloxid absorbiert sind.

Der Gassensor 1 ist heizbar, indem beispielsweise das elektrische Element 2 mit einer elektrischen Energie beaufschlagt wird. Hierzu kann zwischen den Anschlüssen 5 eine Spannungsdifferenz angelegt werden, die zu einem Stromfluss durch das elektrische Element 2 von einem der Anschlüsse 5 zum anderen der Anschlüsse 5 führt. Der Stromfluss wird durch den Widerstand des elektrischen Elements 2 in Wärme umgewandelt. Die so entstehende Wärmeenergie heizt das Metalloxid auf. Die Aufheizung erfolgt unmittelbar in dem räumlichen dreidimensionalen Bereich 7 entlang und zwischen den Elementen 2, 3, der für eine Messung verwendet wird. Eine Aufheizung des Gassensors 1 kann zusätzlich oder alternativ auch durch das andere elektrische Element 3 erfolgen. In weiteren Ausführungsformen können weitere elektrische Elemente zum Heizen und/oder Messen vorgesehen sein. Die Temperaturregelung und Stabilisierung der Heizung kann über die Widerstandsänderung des elektrischen Elements, das einen Heizdraht bildet, erfolgen.

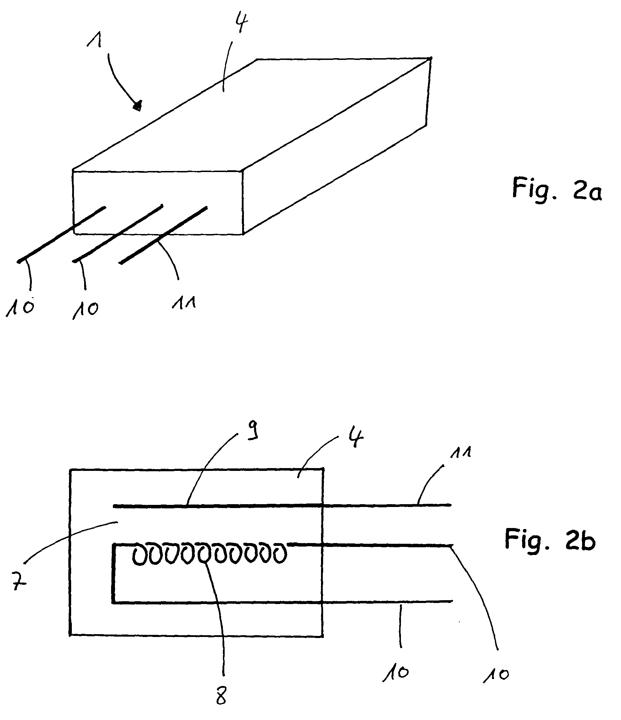

Der in Fig. 2 dargestellte Gassensor 1 weist zwei anders gestaltete elektrische Elemente 8, 9 auf.

Das elektrische Element 8 umfasst einen spiralförmig ausgebildeten Abschnitt und es sind an der Stirnseite des Gassensors 1 zwei Anschlüsse 10 parallel zueinander herausgeführt. Das elektrische Element 8 ist über die beiden Anschlüsse 10 als Heizelement verwendbar, indem es mit einer elektrischen Energie beaufschlagt wird, die in dem spiralförmigen Abschnitt in Wärmeenergie umgesetzt wird. Benachbart zu dem elektrischen Element 8 verläuft ein Abschnitt des elektrischen Elements 9, das über lediglich einen elektrischen Anschluss 11 verfügt. Die Leitfähigkeit im Bereich 7 zwischen den benachbarten Abschnitten der elektrischen Elemente 8, 9 kann in dieser Ausführungsform gemessen werden, indem beispielsweise eine Spannung zwischen den Anschlüssen 10 einerseits und dem Anschluss 11 andererseits angelegt und der Stromfluss zwischen den Anschlüssen 10 und dem Anschluss 11 gemessen wird.

In weiteren Ausführungsformen können die elektrischen Elemente weitere Abschnittsgestaltungen in den einander benachbarten Abschnitten aufweisen. So können die elektrischen Elemente schlicht parallel zueinander verlaufen oder eine helixartige Struktur bilden. Wichtig ist, dass der Abstand so bemessen ist, dass eine Messung bei den angestrebten Betriebstemperaturen sinnvoll möglich ist. Beispielhafte Abstände zwischen den elektrischen Elementen bewegen sich in der Größenordnung von einigen Zehntel Millimetern bis zu 2 mm.

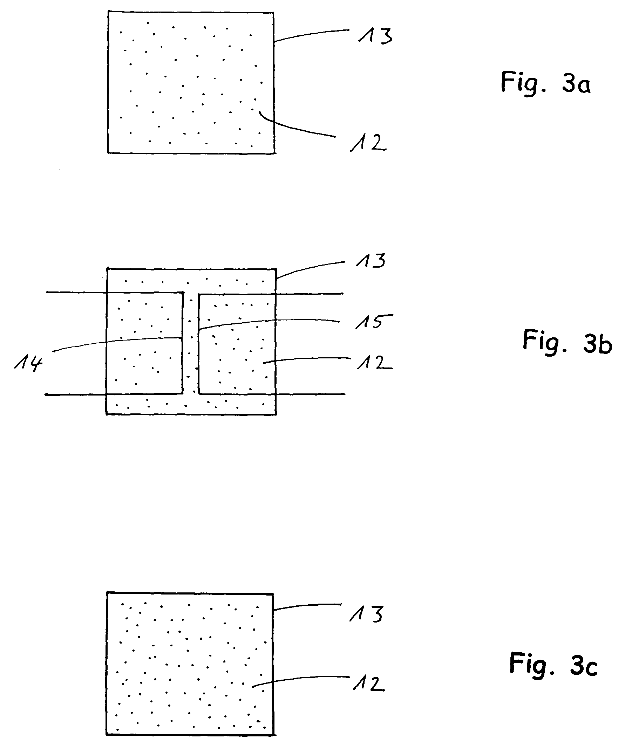

Das in Fig. 3 illustrierte nicht erfindungsgemässe Verfahren zum Herstellen eines Gassensors umfasst im Wesentlichen ein Einfüllen von Metalloxidpulver 12 in eine Form 13, die in Fig. 3(a) in Draufsicht dargestellt ist. Auf diese ggf. flachgedrückte Schicht werden elektrische Elemente, hier beispielhaft zwei elektrische Elemente 14, 15 mit parallel verlaufenden beabstandeten Abschnitten flächig aufgelegt, vgl. Fig. 3(b). Schließlich wird die Form 13 mit Metalloxidpulver 12 zumindest so weit aufgefüllt, dass die elektrischen Elemente 14, 15 vollständig bedeckt sind, vgl. Fig. 3(c). Abschließend kann ggf. nach einem Festdrücken des Metalloxidpulvers ein Sintern durchgeführt werden.

Das Sintern erfolgt vorzugsweise bei Temperaturen oberhalb von 1200°C. Bevorzugte Temperaturbereiche für das Sintern sind 1200°C bis 1600°C und insbesondere 1400°C bis 1500°C. Hierdurch wird die mechanische Festigkeit und die Widerstandsfähigkeit gegenüber Temperaturschwankungen sowie die Korrosionsbeständigkeit des Gassensors erhöht. Es ergibt sich eine poröse Keramik.

Beim Herstellungsverfahren ist sicherzustellen, dass der Abstand zwischen den elektrischen Elementen zumindest abschnittsweise ein gewünschtes Maß verbleibt und eine ausreichende Porosität entsteht. Die Porosität ist erforderlich, damit das zu messende Gas und mit ihm die reduzierenden Stoffe in den Sensor eindringen und mit der Oberfläche insbesondere auch im räumlichen Bereich 7 entlang und zwischen den elektrischen Elementen reagieren können. Die offene Porosität wird hierbei im Bereich zwischen 10 bis 75 % gewählt und liegt bevorzugt bei etwa 40 bis 50 %. Zudem wirkt die offene Porosität Spannungen im Material, die durch thermische Schwankungen entstehen, entgegen.

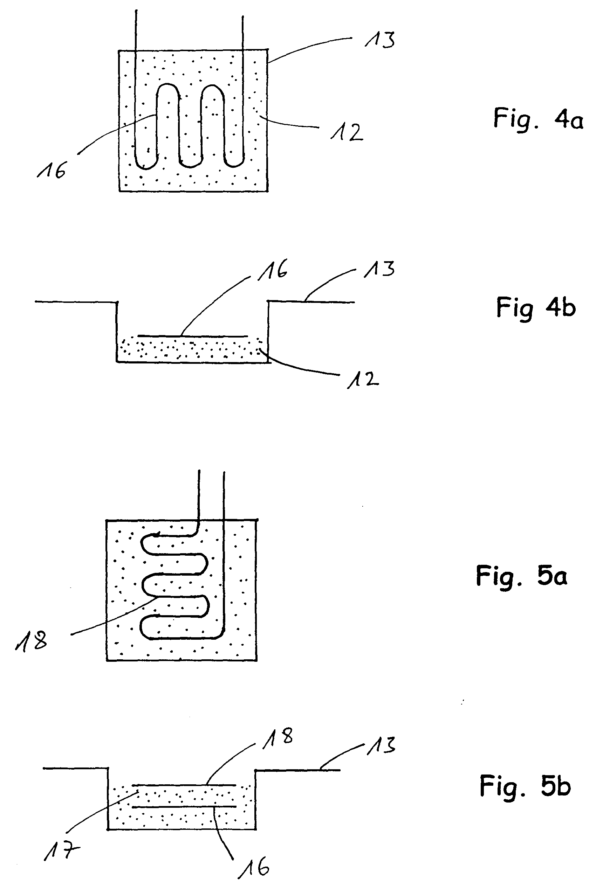

Ein weiteres nicht erfindungsgemässes Herstellungsverfahren wird anhand der Fig. 4 und 5 nunmehr beschrieben.

Demnach wird in eine Form 13 Metalloxidpulver 12 eingebracht und ggf. verfestigt und anschließend ein hier beispielhaft mäanderförmig ausgestaltetes elektrisches Element 16 aufgelegt, vgl. die Draufsicht in Fig. 4(a) auf die Form 13 und den Querschnitt in Fig. 4(b).

Anschließend wird eine Schicht 17, vgl. den Querschnitt durch die Form 13 in Fig. 5(b), aufgebracht und ggf. verdichtet derart, dass die Schicht 17 eine Beabstandung zwischen dem elektrischen Element 16 und einem auf die Schicht 17 aufgelegten weiteren elektrischen Element 18, das hier ebenfalls beispielhaft mäanderförmig dargestellt ist, sicherstellt. Schließlich wird das zweite elektrische Element 18 mit Metalloxidpulver bedeckt und ggf. nach Verfestigen einem Sinterprozess unterzogen.

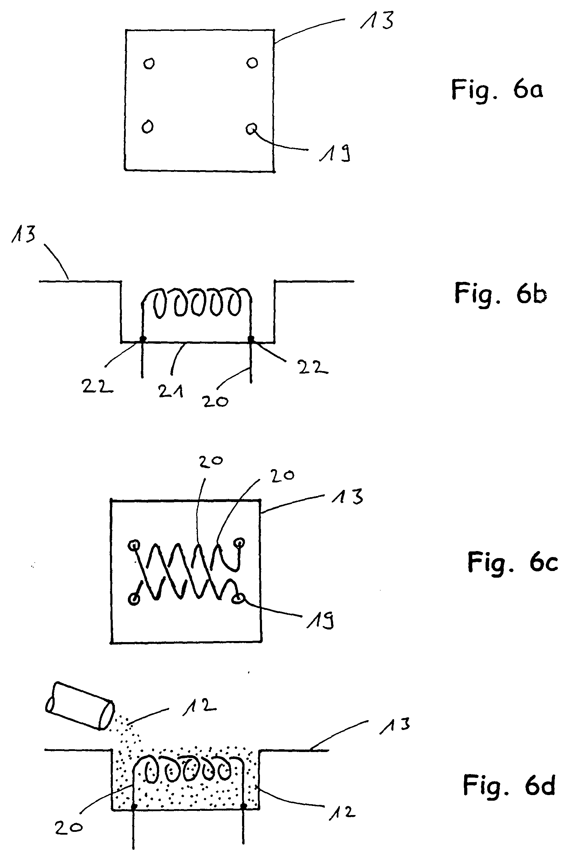

Das in Fig. 6 illustrierte erfindungsgemässe zur Herstellung des Gassensors verwendet eine Fülltechnik. Zunächst werden in eine leere Form 13 mit vier bodenseitigen Kontaktdurchgängen 19, vgl. die Draufsicht in Fig. 6(a), elektrische Elemente 20 dergestalt eingesetzt, dass die elektrischen Elemente zueinander und auch zum Boden 21 der Form 13 zweckmäßig beabstandet sind, vgl. den Querschnitt und die Draufsicht in Fig. 6(b) bzw. (c).

Die elektrischen Elemente 20 sind hier beispielhaft spulenförmig dargestellt mit einer Spulenachse, die parallel zur Längsachse des hier beispielhaft quaderförmigen Gassensors verläuft. Die elektrischen Elemente 20 erstrecken sich dabei jedenfalls im Bereich der Spulen vorzugsweise parallel zueinander in den vorstehend genannten zweckmäßigen Abständen. Der Abstand wird sichergestellt durch eine Hilfskonstruktion wie etwa ein kammartig von oben in die Spulenwindungen eingreifendes Abstandselement.

Der Abstand zum Boden 21 der Form 13 kann beispielsweise durch punktuelles Zusammendrücken der elektrischen Elemente 20 zwecks Ausbildung verbreiteter Bereiche 22 sichergestellt werden, die breiter sind als die Durchmesser der Kontaktdurchgänge 19.

Nach Positionierung der elektrischen Elemente 20 in der Form 13 kann die Form 13 in einem Schritt mit Metalloxid gefüllt werden, vgl. Fig. 6(d). Abschließend kann ggf. unter Druck gesintert werden.

Das Metalloxidpulver, beispielsweise Galliumoxidpulver, kann eine Korngröße von etwa 10 µm aufweisen.

In weiteren Ausführungsformen werden die Gassensoren mittels Gieß-, Spritzgussund Foliengussverfahren hergestellt, die allesamt mit den vorgenannten Verfahren insbesondere der Figuren 3 bis 6 verwendbar sind mit der Maßgabe, dass das Metalloxidpulver (MOX) in Form einer Mischung zugeführt wird.

In einer Ausführungsform wird der Gassensor mittels eines Gießverfahrens hergestellt. Die MOX Pulvermischung wird mittels Wasser, Dispergiermittel und Bindern in einem geeigneten Dispergierer zu einem keramischen Schlicker verarbeitet. Ein gängiges Rezept für den Giesschlicker besteht aus:
100g MOX Pulvermischung
20 - 30g Wasser (Dispergiermedium)
0,3 - 0,9g Diammoniumcitrat (Dispergiermittel)
0,01 - 0,03g Methylcelulose (Binder)

Der Schlicker wird in die Form, in der sich die Elektroden bzw. elektrischen Elemente befinden, gegossen. Nach dem Aushärten und Trocknen wird der der Grünling entformt und nachgearbeitet.

In einer weiteren Ausführungsform wird der Gassensor in einem Spritzgussverfahren hergestellt. Hierzu wird eine MOX Pulvermischung mittels Binder und Additiven zu einem Granulat verarbeitet. Dieses Granulat wird bis zum Erweichungs/Schmelzpunkt der Binder erwärmt und heiß in die Form in der sich bereits die elektrischen Elemente befinden gepresst (Injection Molding). Ein übliches Rezept dafür besteht aus:
100g MOX Pulvermischung
20 - 40g Polycrylsäure (PMMA)
5 - 10g Oleinsäure

Schließlich kann der Gassensor auch mit einem Foliengussverfahren hergestellt werden. Hierzu wird die MOX Pulvermischung mittels Alkohol, Dispergiermittel, Plastifiziermitteln und Bindern in einem geeigneten Dispergierer zu einem keramischen Schlicker verarbeitet. Ein gängiges Rezept für den Foliengießschlicker besteht aus:
100g MOX Pulvermischung
40 - 60g Ethylen (Dispergiermedium)
10 - 14g Dimethyphtalat (Plastifizierer)
10 - 20g Ethylcelulose (Binder)

Der Schlicker wird in einen Trichter eingefüllt, an dessen unteren Ende sich ein Spalt befindet, der die Höhe und Breite des Substrates definiert. Der Schlicker wird durch den Spalt auf eine Kunststofffolie ausgegossen. Die Folie ist auf einer Rolle gelagert und wird unterhalb des Spaltes entlanggezogen. Es bildet sich eine plastische keramische Folie. In diese werden die elektrischen Elemente eingetaucht. Nach dem Trocknen der keramischen Folie wird die für den Gassensor benötigte Form ausgeschnitten oder ausgestanzt.

In allen Ausführungsformen wird abschließend ein Sinterprozess durchgeführt.

In den dargestellten Ausführungsformen sind die Anschlüsse 5, 6, 10, 11 jeweils seitlich oder nach unten aus dem Gassensor 1 herausgeführt. In weiteren Ausführungsformen können andere Kontaktierungen zu den elektrischen Elementen 2, 3, 8, 9, 14, 15, 16, 18 vorgesehen sein beispielsweise in Form von freiliegenden Kontaktflächen.

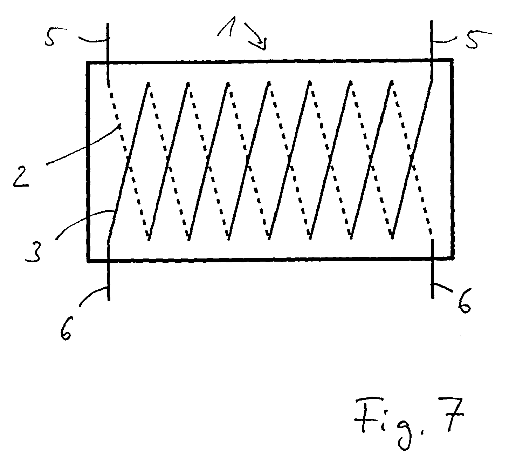

In Fig. 7 ist eine weitere erfindungsgemässe Ausführungsform mit zwei helixartig gewundenen elektrischen Elementen 2, 3 dargestellt. Die gestrichelte Linie ist in der dargestellten Draufsicht jeweils räumlich unten, während die durchgezogene Linie sich jeweils räumlich oben befindet. Die beiden spiralförmig gewundenen Elemente 2, 3 sind idealerweise stets gleichbeabstandet von einander. Hierzu kann ein Stützmittel verwendet werden, wie etwa eine Kammstruktur oder die Elemente 2, 3 können eingebettet sein in Wachs oder dergleichen. Das Stützmittel kann bei der gemeinsamen Herstellung der beiden Elemente 2, 3 oder beim Zusammenfügen der getrennt hergestellten Elemente 2, 3 verwendet werden und vor dem Einlegen in eine Form oder danach und ggf. während oder nach Einbetten in Metalloxid entfernt werden oder ggf. auch permanent verbleiben.

### BEZUGSZEICHENLISTE

- 1: Gassensor
- 2, 3, 8, 9, 14, 15, 16, 18, 20: elektrisches Element
- 4: Metalloxid
- 5, 6, 10, 11: Anschluss
- 7: Bereich
- 12: Metalloxidpulver
- 13: Form
- 17: Schicht
- 19: Kontaktdurchgänge
- 21: Boden
- 22: verbreiterte Bereiche

## Patentansprüche

1. Verwendung eines Stützmittels zum Sicherstellen einer dreidimensionalen Struktur aus elektrischen Elementen bei der Herstellung eines heizbaren Gassensors (1) mit einem gassensitiven porösen Metalloxid (4) sowie voneinander beabstandeten und jeweils räumlich im gesinterten Metalloxid (4) eingebetteten elektrischen Elementen (2, 3, 20) nach dem Verfahren nach einem der Ansprüche 2 bis 7, **dadurch gekennzeichnet, dass** das Stützmittel eine Kammstruktur oder Wachs aufweist, die zum Sicherstellen des Abstands zwischen zwei helixartig um eine gemeinsame Achse gewundenen elektrischen Elementen (2, 3, 20), die zum Heizen des Metalloxids (4) des Gassensors (1) und zum Messen einer Leitfähigkeit des Metalloxids (4) des Gassensors (1) ausgestaltet sind, in die Windungen der elektrischen Elemente eingreift.

2. Verfahren zum Herstellen eines heizbaren Gassensors (1) für reduzierende Stoffe enthaltende Gase **dadurch gekennzeichnet, dass** wenigstens zwei helixartig um eine gemeinsame Achse gewundene elektrische Elemente (2, 20) unter Verwendung eines Stützmittels (19) zueinander beabstandet angeordnet und gemeinsam in gassensitivem porösem Metalloxid (4) räumlich eingebettet werden und das Metalloxid (4) gesintert wird, wobei das Stützmittel (19) eine Kammstruktur oder Wachs umfasst.

3. Verfahren nach Anspruch 2, **dadurch gekennzeichnet, dass** das Stützmittel (19) vor oder nach dem Einbetten in Metalloxid (4) entfernt wird.

4. Verfahren nach einem der Ansprüche 2 oder 3, **dadurch gekennzeichnet, dass** die elektrischen Elemente (2, 3, 20) in Metalloxidpulver (12) eingebettet werden.

5. Verfahren nach einem der Ansprüche 2 bis 4, **dadurch gekennzeichnet, dass** das Metalloxidpulver (12) mit den eingebetteten elektrischen Elementen (2, 3, 20) gesintert wird.

## Claims

1. Use of a support structure for securing a three-dimensional structure formed of electrical elements in the production of a heatable gas sensor (1) with a gas-sensitive porous metal oxide (4) and electrical elements (2, 3, 20) that are spaced apart from one another and each spatially embedded in the sintered metal oxide (4), according to the method according to any one of Claims 2 to 7, **characterized in that** said support structure has a comb structure or wax, which in order to secure the spacing between two helical electrical elements (2, 3, 20) wound around a common axis which are designed for heating the metal oxide (4) of the gas sensor (1) and for measuring a conductivity of the metal oxide (4) of the gas sensor (1), engages with the windings of the electrical elements.

2. Method for producing a heatable gas sensor (1) for gases containing reducing materials, **characterized in that** at least two electrical elements (2, 20) helically wound around a common axis are spaced apart from each other using a support structure (19) and spatially embedded together in gas-sensitive porous metal oxide (4) and the metal oxide (4) is sintered, wherein the support structure (19) comprises a comb structure or wax.

3. Method according to Claim 2, **characterized in that** the support structure (19) is removed before or after the embedding in metal oxide (4).

4. Method according to any one of Claims 2 or 3, **characterized in that** the electrical elements (2, 3, 20) are embedded in metal oxide powder (12).

5. Method according to any one of Claims 2 to 4, **characterized in that** the metal oxide powder (12) is sintered with the embedded electrical elements (2, 3, 20).

## Revendications

1. Utilisation d'une structure de support pour fixer de manière sécurisée une structure tridimensionnelle composée d'éléments électriques lors de la fabrication d'un capteur de gaz (1) pouvant être chauffé avec un oxyde métallique (4) poreux sensible aux gaz ainsi que des éléments électriques (2, 3, 20) espacés les uns des autres et respectivement incorporés spatialement dans l'oxyde métallique fritté (4) selon le procédé selon une des revendications 2 à 7, **caractérisé en ce que** la structure de support présente une structure de peigne ou en cire, qui pour garantir l'espacement entre deux éléments électriques (2, 3, 20) en forme d'hélice et enroulés autour d'un axe commun, qui sont conçus pour chauffer l'oxyde métallique (4) du capteur de gaz (1) et pour mesurer une conductivité de l'oxyde métallique (4) du capteur de gaz (1), vient en prise dans les spires des éléments électriques.

2. Procédé de fabrication d'un capteur de gaz (1) pouvant être chauffé pour des gaz contenant des matières réductrices, **caractérisé en ce que** au moins deux éléments électriques (2, 20) en forme d'hélice enroulés autour d'un axe commun sont disposés en espacement les uns par rapport aux autres en utilisant une structure d'appui (19) et sont incorporés spatialement en commun dans un oxyde métallique (4) poreux sensible aux gaz et l'oxyde métallique (4) est fritté, dans lequel la structure de support (19) comprend une structure de peigne ou en cire.

3. Procédé selon la revendication 2, **caractérisé en ce que** la structure de support (19) est retirée avant ou après l'incorporation dans l'oxyde métallique (4).

4. Procédé selon une des revendications 2 ou 3, **caractérisé en ce que** les éléments électriques (2, 3, 20) sont incorporés dans une poudre d'oxyde métallique (12).

5. Procédé selon une des revendications 2 à 4, **caractérisé en ce que** la poudre d'oxyde métallique (12) est frittée avec les éléments électriques incorporés (2, 3, 20).
